# EUROPÄISCHE PATENTSCHRIFT

(11) **EP 0 285 681 B1**
(45) Veröffentlichungstag und Bekanntmachung des Hinweises auf die Patenterteilung: **10.03.1993**
(21) Anmeldenummer: 87105064.7
(22) Anmeldetag: 06.04.1987
(51) Int. Cl.: C07D 307/52, C07D 277/28

(54) **Verfahren zur Herstellung von Nitroethenderivaten**
Process for the preparation of nitroethylene derivatives
Procédé pour la préparation de dérivés de nitroéthène

(43) Veröffentlichungstag der Anmeldung: 12.10.1988
(73) Patentinhaber: HEUMANN PHARMA GMBH & CO, D-90478 Nürnberg (DE)
(72) Erfinder: Mörsdorf, Dr. Peter, Dipl. Chem., D-8506 Langenzenn (DE); Schickaneder, Dr. Helmut, Dipl.Chem., D-8501 Eckental (DE); Ahrens, Dr. Kurt Henning, D-8500 Nürnberg (DE)
(74) Vertreter: Kraus, Walter, Dr.

(56) Entgegenhaltungen:
- EP-A- 0 049 618
- DE-A- 2 423 813
- DE-A- 2 734 070
- US-A- 4 128 658
- CHEMICAL ABSTRACTS, Band 87, Nr. 25, 19. Dezember 1977, Seite 683, Spalte 2, Zusammenfassungsnr. 201032g, Columbus, Ohio, US; V.V. RUDCHENKO et al.: Synthesis and structure of nitroacetamidine derivatives" & ZH. ORG. KHIM. 1977, 13(7), 1383-1386
- CHEMICAL ABSTRACTS, Band 90, Nr. 13, 26. März 1979, Seite 5, Spalte 2, Zusammenfassungsnr. 103308u, Columbus, Ohio, US; F.E. VERBRUGGEN et al.: "Substitution in mono-, di-, and trichloronitroethylenes. Syntheses of derivatives of nitroacetaldehyde and nitro- and chloronitroacetic acids"; & BULLITIN DE SOCIETE CHIMIQUE BELGE 1978, 87(9), 693-707
- Houben-Weyl, Methoden der Organischen Chemie E5, 812/813
- Chem. Ber. 100, 2604(1967)
- V.A. Buerich et al., Zhurnal Organicheskoi Khimii 17, 1550-1551 (1980)

## Beschreibung

Die Erfindung betrifft ein neues Verfahren zur Herstellung von Nitroethenderivaten, insbesondere von N-[2-[[[5-[(Dimethylamino)methyl]-2-furanyl]methyl]thio]ethyl]-Nʹ-methyl-2-nitro-1,1-ethendiamin und N-[2-[[[2-[(Dimethylamino)methyl]-4-thiazolyl]methyl]thio]ethyl]-Nʹ-methyl-2-nitro-1,1-ethendiamin und deren physiologisch annehmbarer Salze. Diese als Ranitidin (INN) und Nizatidin (INN) bezeichneten Verbindungen sind bereis aus den DE-OSʹen 2 734 070 und 3 521 456 und der EP-OS 0 049 618 bekannt.

Die vorliegende Erfindung betrifft auch die neuen Verbindungen 1-[2-[[[5-[(Dimethylamino)methyl]-2-furanyl]methyl]thio]ethylamino]-2-nitro-1-phenoxyethen und 1-[2-[[[2-[(Dimethylamino)methyl]-4-thiazolyl]methyl]-thio]ethylamino]-2-nitro-1-phenoxyethen und ihre Salze, die Zwischenprodukte bei dem Verfahren gemäß der vorliegenden Erfindung darstellen.

Ranitidin und Nizatidin sind H₂-Rezeptorenblocker und werden bei der Behandlung von Magengeschwüren zur Hemmung der histaminstimulierten Magensäuresekretion eingesetzt.

Zur Herstellung von Ranitidin sind verschiedene Verfahren bekannt. Von industrieller Bedeutung sind jedoch hauptsächlich die in den DE-OSʹen 2 734 070 und 3 521 456 beschriebenen Umsetzungen von 2-[[[5-[(Dimethylamino)methyl]-2-furanyl]methyl]thio]ethylamin mit N-Methyl-1-methylthio-2-nitroethenamin bzw. das zweistufige Verfahren der Umsetzung von 2-[[[5-[(Dimethylamino)methyl]-2-furanyl]methyl]thio]ethylamin zunächst mit 1,1-Bis-(methylthio)-2-nitroethen und dann mit Methylamin.

Nizatidin wird nach der EP-OS 0 049 618 nach analogen Verfahren aus 2-[[[2-[(Dimethylamino)methyl]-4-thiazolyl]methyl]thio]ethylamin und N-Methyl-1-methylthio-2-nitroethenamin bzw. 1,1-Bis(methylthio)-2-nitroethen und Methylamin hergestellt.

Der Erfindung liegt die Aufgabe zugrunde, ein neues, verbessertes Verfahren zur Herstellung von Ranitidin und Nizatidin zur Verfügung zu stellen, welches insbesondere die Umweltbelastungen der bisherigen Verfahren durch Freisetzung von Methylmercaptan vermeidet.

Diese Aufgabe wird durch die Erfindung gelöst.

Gegenstand der Erfindung is daher ein Verfahren zur Herstellung von Nitroethenderivaten der allgemeinen Formel I 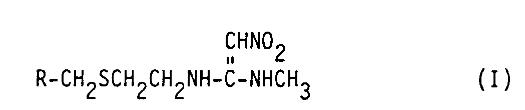
in der R für die Gruppe 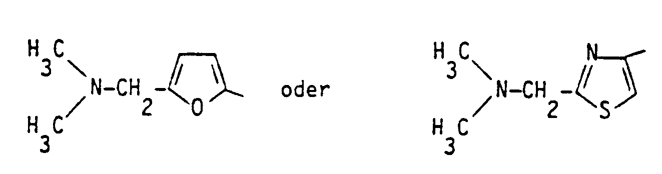
steht, oder deren physiologisch annehmbarer Salze, das dadurch gekennzeichnet ist, daß man
a) ein Amin der allgemeinen Formel II

   R-CH₂SCH₂CH₂NH₂ (II)

   in der R die oben angegebene Bedeutung hat, mit einer Verbindung der allgemeinen Formel IV 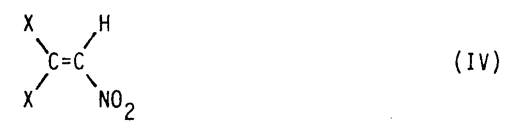 in der X für eine gegebenenfalls substituierte Phenoxygruppe steht, zu einer Zwischenverbindung der allgemeinen Formel V 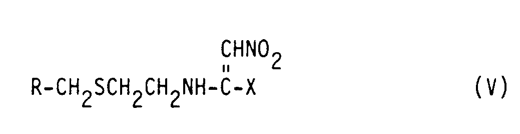 in der R und X die oben angegebenen Bedeutungen haben, umsetzt,
b) die erhaltene Zwischenverbindung der allgemeinen Formel V mit Methylamin zu einer Verbindung der allgemeinen Formel I umsetzt
   und gegebenenfalls
c) die erhaltene Verbindung der allgemeinen Formel I in an sich bekannter Weise in ein physiologisch annehmbares Salz umwandelt.

Im folgenden wird das erfindungsgemäße Verfahren näher beschrieben:
1. In der ersten Stufe des erfindungsgemäßen Verfahrens wird ein Amin der allgemeinen Formel II

   R-CH₂SCH₂CH₂NH₂ (II)

   in der R die oben angegebene Bedeutung hat, mit einer Verbindung der allgemeinen Formel IV 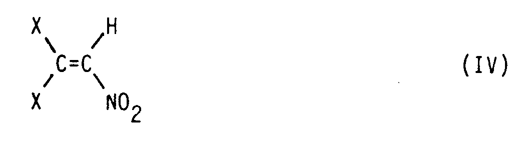 in der X für eine gegebenenfalls substituierte Phenoxygruppe steht, in einem geeigneten Lösungsmittel umgesetzt.
   In der allgemeinen Formel IV steht X für oder eine Phenoxygruppe, die gegebenenfalls substituiert sein kann. Hierbei kommen als Substituenten für die Phenoxygruppe vorzugsweise ein oder mehrere, insbesondere ein bis drei, Halogenatome, wie Chloroder Bromatome, C₁-C₄-Alkylgruppen, C₁-C₄-Alkoxygruppen oder Nitrogruppen in Betracht. Besonders bevorzugte Ausgangsverbindungen der allgemeinen Formel IV ist 1,1-Diphenoxy-2-nitroethen (IVb) mit den folgenden Strukturformeln: 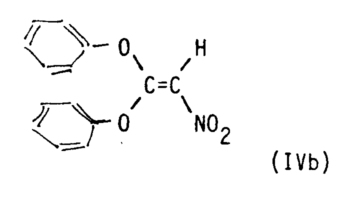
   Als Lösungsmittel können zum Beispiel Ether, wie Tetrahydrofuran oder Dioxan, Ketone, wie Aceton, Methylethylketon oder 4-Methyl-2-pentanon, sowie Acetonitril Verwendung finden. Die Reaktionstemperatur wird zweckmäßig zwischen Raumtemperatur, zum Beispiel 20°C, und dem Siedepunkt des verwendeten Lösungsmittels gehalten. Das Mengenverhältnis der Ausgangsverbindungen liegt im Bereich von 2:1 bis 1:1, vorzugsweise 1:1.
   Das so erhaltene Zwischenprodukt der allgemeinen Formel V kann nach an sich bekannten Verfahren isoliert, gereinigt und dann in einer zweiten Stufe zum Endprodukt der Formel I umgesetzt werden. Es ist aber auch möglich, auf die Isolierung des Zwischenprodukts der allgemeinen Formel V zu verzichten und beide Reaktionsstufen in einem Eintopfverfahren durchzuführen.
   Die als Zwischenprodukte in der ersten Stufe des erfindungsgemäßen Verfahrens entstehenden Verbindungen 1-[2-[[[5-(Dimethylamino)methyl]-2-furanyl]methyl]thio]ethylamino]-2-nitro-1-phenoxyethen der Formel Va und 1-[2-[[[2-[(Dimethylamino)methyl]-4-thiazolyl]methyl]thio]ethylamino]-2-nitro-1-phenoxyethen der Formel Vb sind neue Verbindungen. Sie lassen sich auch als Säureadditionsprodukte herstellen. Sie und ihre Salze sind daher ebenfalls ein Gegenstand der vorliegenden Erfindung. 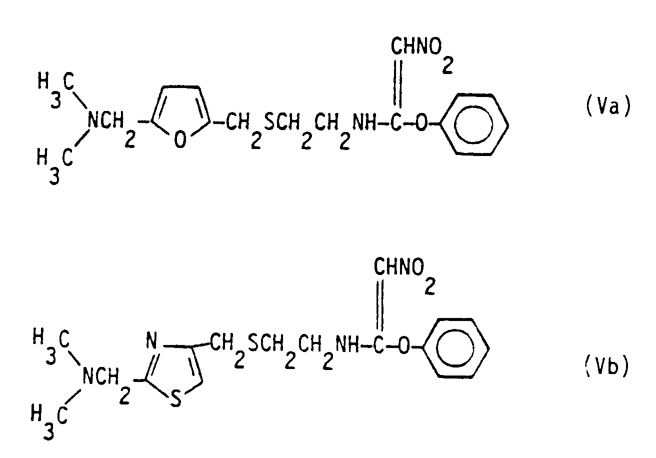
2. In der zweiten Stufe des erfindungsgemäßen Verfahrens setzt man die Verbindung der allgemeinen Formel V mit überschüssigem Methylamin quantitativ zu N-[2-[[[5-[(Dimethylamino)methyl]-2-furanyl]methyl]thiolethyl]-Nʹ-methyl-2-nitro-1,1-ethendiamin bzw. N-[2-[[[2-[(Dimethylamino)methyl]-4-thiazolyl]methyl]thio]ethyl]-Nʹ-methyl-2-nitro-1,1-ethendiamin um. Die Umsetzung wird beispielsweise in einem Ether, einem Alkohol oder Wasser als Lösungsmittel durchgeführt. Beispiele für geeignete Alkohole sind Methanol, Ethanol, Isopropanol oder n-Butanol. Als Ether werden Tetrahydrofuran und Dioxan bevorzugt. Das Methylamin kann gasförmig oder als Lösung in dem bei der Reaktion verwendeten Lösungsmittel eingesetzt werden und wird im Oberschuß, beispielsweise im Molverhältnis 2:1 bis 10:1, vorzugsweise 5:1, bezogen auf das Zwischenprodukt der allgemeinen Formel V, angewendet. Die Reaktionstemperatur kann zwischen 0°C und dem Siedepunkt des verwendeten Lösungsmittels liegen, wobei Temperaturen von 20 bis 30°C bevorzugt werden. Die Isolierung der auf diese Weise hergestellten Verbindungen der Formel I erfolgt nach allgemein bekannten Verfahrensweisen, beispielsweise durch Kristallisation aus einem geeigneten Lösungsmittel etc.

Die in der zweiten Stufe des erfindungsgemäßen Verfahrens erhaltene Verbindung der allgemeinen Formel I, d.h. das Ranitidin oder das Nizatidin, kann in an sich bekannter Weise in ihr physiologisch annehmbares Salz umgewandelt werden. Das Salz kann sich zum Beispiel von einer Mineralsäure, wie Chlor-, Brom- oder Jodwasserstoffsäure, Phosphorsäure, Metaphosphorsäure, Salpetersäure oder Schwefelsäure, oder von einer organischen Säure, wie Ameisensäure, Essigsäure, Propionsäure, Phenylessigsäure, Weinsäure, Zitronensäure, Fumarsäure, Methansulfonsäure etc., ableiten.

Die nach dem erfindungsgemäßen Verfahren erhaltenen Verbindungen können für die Verabreichung in der gleichen Weise formuliert werden, wie es für Ranitidin und Nizatidin und deren Salze, insbesondere die Hydrochloride, bereits bekannt ist.

Die Hauptvorteile des erfindungsgemäßen Verfahrens liegen einerseits in einer höheren Reinheit des erhaltenen Produkts. Andererseits liegen sie vor allem in einer besseren Ökologie des Verfahrens, da im Gegensatz zu den bisher bekannten Herstellungsmethoden für Ranitidin und Nizatidin bei dem erfindungsgemäßen Verfahren kein hochtoxisches und übelriechendes Methylmercaptan entsteht.

Die Erfindung wird in den Beispielen erläutert.

### Beispiel 1

### a) 1-[2-[[[5-[(Dimethylamino)methyl]-2-furanyl]methyl]thio]ethylamino]-2-nitro-1-phenoxyethen

Eine Lösung von 2,14 g (10 mmol) 2-[[[5-[(Dimethylamino)methyl]-2-furanyl] methyl]thio]ethylamin und 2,57 g (10 mmol) 1,1-Diphenoxy-2-nitroethen in 20 ml Acetonitril wird 2 Stunden gekocht. Nach Eindampfen der Lösung im Vakuum wird der ölige Rückstand - wie in Bsp. 1 beschrieben - chromatographisch gereinigt und ergibt 3,06 g (81 %) der Titelverbindung als blaßgelbes Öl, nach DC und ¹H-NMR-Spektrum identisch mit der Verbindung aus Beispiel 1.

### b) N-[2-[[[5-[(Dimethylamino)methyl]-2-furanyl]methyl]thio]ethyl]-Nʹ-methyl-2-nitro-1,1-ethendiaminhydrochlorid

Zu einer Lösung von 37,7 g (0.1 mol) 1-[2-[[[5-[(Dimethylamino)methyl]-2-furanyl]methyl]thio]ethylamino]-2-nitro-1-phenoxy-ethen in 100 ml Methanol werden 45 ml (0.5 mol) einer Lösung von Methylamin in Methanol (11 mol/l) bei Raumtemperatur getropft. Nach dreistündigem Rühren wird die Lösung i. Vak. eingedampft und der Rückstand in 100 ml Ethanol aufgenommen. Die erhaltene Lösung wird zunächst mit 25 ml einer Lösung von Chlorwasserstoff in Ethanol (4 mol/l) und dann langsam mit 125 ml Ethylacetat versetzt. Das ausgefallene Hydrochlorid wird abgesaugt, mit 30 ml Ethylacetat gewaschen und im Vakuum getrocknet.
31,9 g (91 %) farblose Kristalle vom Schmp. 133 - 134° C.

### Beispiel 2

### N-[2-[[[2-[(Dimethylamino)methyl]-4-thiazolyl]methyl]thio]ethyl]-Nʹ-methyl-2-nitro-1,1-ethendiamin

Eine Lösung von 2,37 g (6 mmol) 1-[2-[[[2-[(Dimethylamino)methyl]-4-thiazolyl]methyl]thio]ethylamino]-2-nitro-1-phenoxyethen, das analog zu Beispiel 1a) erhalten wurde, in 10 ml Methanol wird bei Raumtemperatur mit 3 ml einer Lösung von Methylamin in Methanol (11 mol/l) versetzt und 3,5 Stunden gerührt. Die Lösung wird dann im Vakuum eingedampft und der Rückstand mit 5 ml Ethylacetat/Ethanol (2:1) versetzt. Nach zehnminütigem Rühren wird der Feststoff abgesaugt und im Vakuum getrocknet. 2,99 g (90 %) farblose Kristalle vom Schmp. 131 - 132° C.

## Patentansprüche

1. Verfahren zur Herstellung von Nitroethenderivaten der allgemeinen Formel I 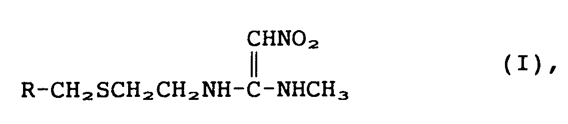 in der R für die Gruppe 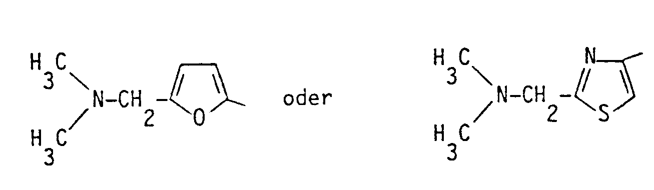 steht, oder deren physiologisch annehmbarer Salze, dadurch **gekennzeichnet,** daß man
a) ein Amin der allgemeinen Formel II
R-CH₂SCH₂CH₂NH₂ (II),
in der R die oben angegebene Bedeutung hat, mit einer Verbindung der allgemeinen Formel IV 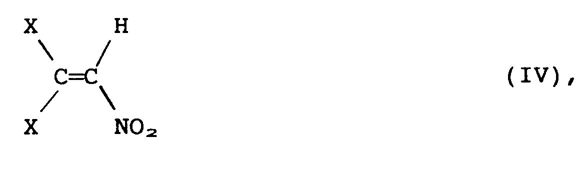 in der X für eine gegebenenfalls substituierte Phenoxygruppe steht, zu einer Zwischenverbindung der allgemeinen Formel V 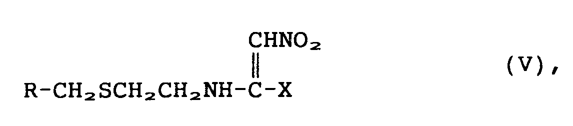 in der R und X die oben angegebenen Bedeutungen haben, umsetzt,
b) die erhaltene Zwischenverbindung der allgemeinen Formel V mit Methylamin zu einer Verbindung der allgemeinen Formel I umsetzt
und gegebenenfalls
c) die erhaltene Verbindung der allgemeinen Formel I in an sich bekannter Weise in ein physiologisch annehmbares Salz umwandelt.

2. Verfahren nach Anspruch 1, dadurch **gekennzeichnet,** daß die eingesetzte Verbindung der allgemeinen Formel IV 1,1-Diphenoxy-2-nitroethen ist.

3. Verfahren nach Anspruch 1, dadurch **gekennzeichnet,** daß man die Stufen a) und b) in einem "Eintopfverfahren" durchführt.

4. 1-[2-[[[5-[(Dimethylamino)methyl]-2-furanyl]methyl]thio]ethylamino]-2-nitro-1-phenoxyethen der Formel Va

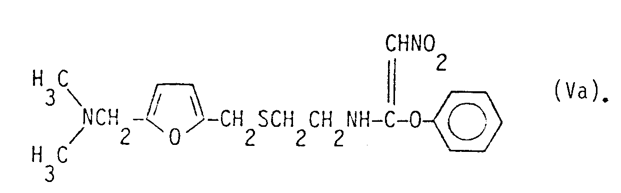

5. 1-[2-[[[2-[(Dimethylamino)methyl]-4-thiazolyl]methyl]thio]ethylamino]-2-nitro-1-phenoxyethen der Formel Vb

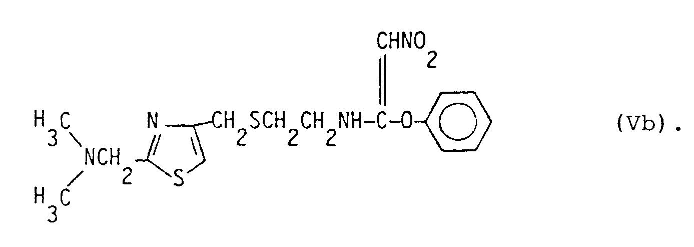

## Claims

1. A process for the production of nitroethene derivatives corresponding to general formula I 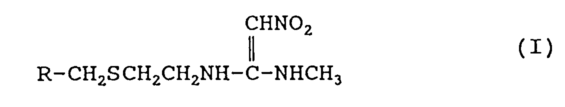 in which R represents the group 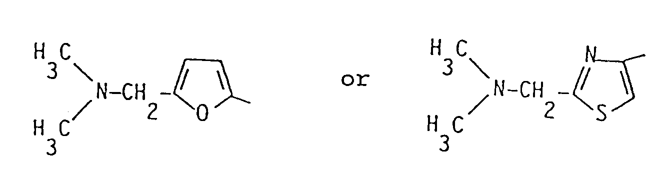 or physiologically acceptable salts thereof, characterized in that
a) an amine corresponding to general formula II
R-CH₂SCH₂CH₂NH₂ (II)
in which R is as defined above,
is reacted with a compound corresponding to general formula IV 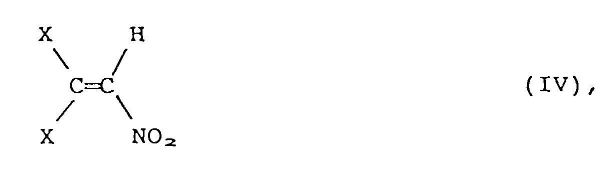 in which X is an optionally substituted phenoxy group, to form an intermediate compound corresponding to general formula V 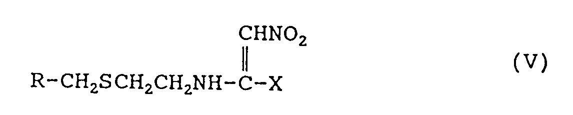 in which R and X are as defined above,
b) the intermediate compound corresponding to general formula (V) is reacted with methylamine to form a compound corresponding to general formula I
and, optionally,
c) the resulting compound of general formula (I) is converted into a physiologically acceptable salt by methods known per se.

2. A process as claimed in claim 1, characterized in that the compound of general formula IV used is 1,1-diphenoxy-2-nitroethene.

3. A process as claimed in claim 1, characterized in that steps a) and b) are carried out as a "one-pot process".

4. 1-[2-[[[5-(dimethylamino)methyl]-2-furanyl]methyl]thio]ethylamino]-2-nitro-1-phenoxyethene corresponding to formula Va

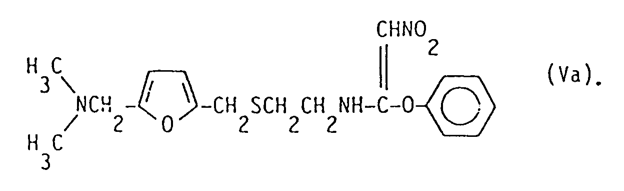

5. 1-[2-[[[2-(dimethylamino)methyl]-4-thiazolyl]methyl]thio]ethylamino]-2-nitro-1-phenoxyethene corresponding to formula Vb

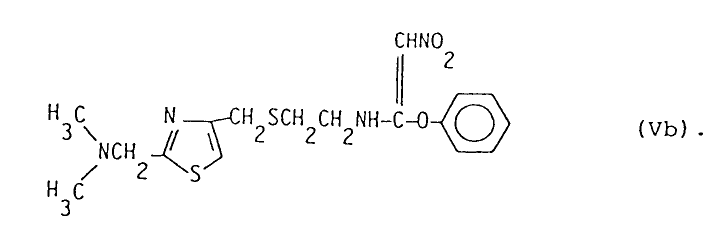

## Revendications

1. Procédé pour la fabrication de dérivés de nitroéthylène de formule générale I : 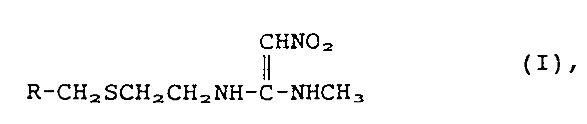 dans laquelle R représente le groupe : 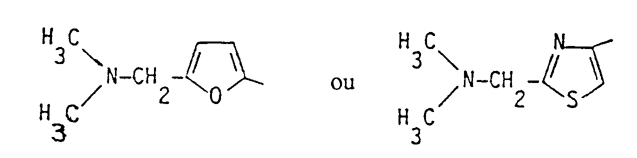 ou de leurs sels physiologiquement acceptables, caractérisé en ce que :
a) on fait réagir une amine de formule générale II
R-CH₂SCH₂CH₂NH₂ (II)
dans laquelle R a la signification indiquée ci-dessus, avec un composé de formule générale IV : 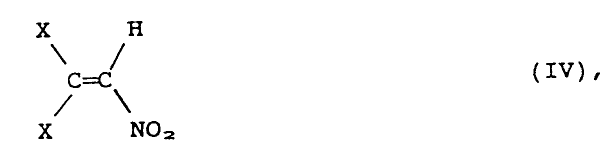 dans laquelle X représente un groupe phénoxy éventuellement substitué, pour donner un composé intermédiaire de formule générale V : 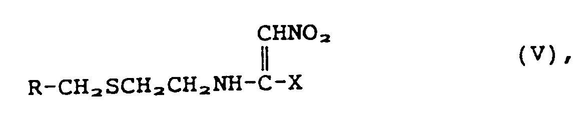 dans laquelle R et X ont les significations indiquées ci-dessus,
b) on fait réagir le composé intermédiaire de formule générale V obtenu avec la méthylamine pour donner un composé de formule générale I
et éventuellement
c) on transforme de manière connue le composé de formule générale I obtenu en un sel physiologiquement acceptable.

2. Procédé selon la revendication 1, caractérisé en ce que le composé de formule générale IV utilisé est le 1,1-diphénoxy-2-nitroéthylène.

3. Procédé selon la revendication 1, caractérisé en ce que l'on met en oeuvre les étapes a) et b) dans un procédé en un pot.

4. Le 1-[2-[[[5-[(diméthylamino)méthyl]-2-furyl]méthyl]thio]éthylamino]-2-nitro-1-phénoxyéthylène de formule Va :

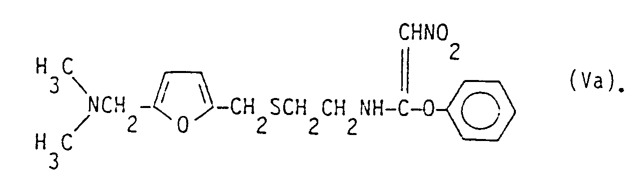

5. Le 1-[2-[[[2-[(diméthylamino)méthyl]-4-thiazolyl]méthyl]thio]éthylamino]-2-nitro-1-phénoxyéthylène de formule Vb :
